# EUROPÄISCHE PATENTSCHRIFT

(11) **EP 0 739 403 B1**
(45) Veröffentlichungstag und Bekanntmachung des Hinweises auf die Patenterteilung: **09.06.1999**
(21) Anmeldenummer: 95903794.6
(22) Anmeldetag: 06.12.1994
(51) Int. Cl.: C09K 19/34, C09K 19/32, C09K 19/30, C09K 19/20

(54) **POLYMERISIERBARE CHIRALE VERBINDUNGEN UND DEREN VERWENDUNG**
POLYMERIZABLE CHIRAL COMPOUNDS AND THEIR USE
COMPOSES CHIRAUX POLYMERISABLES ET LEUR UTILISATION

(30) Priorität: 11.12.1993 DE 4342280
(43) Veröffentlichungstag der Anmeldung: 30.10.1996
(73) Patentinhaber: BASF AKTIENGESELLSCHAFT, 67056 Ludwigshafen (DE)
(72) Erfinder: ETZBACH, Karl-Heinz, D-67227 Frankenthal (DE); DELAVIER, Paul, D-67061 Ludwigshafen (DE); SIEMENSMEYER, Karl, D-67227 Frankenthal (DE); WAGENBLAST, Gerhard, D-67157 Wachenheim (DE); LAUPICHLER, Lothar, D-69121 Heidelberg (DE); VILL, Volkmar, D-20255 Hamburg (DE)
(86) Internationale Anmeldenummer: EP9404055
(87) Internationale Veröffentlichungsnummer: WO9516007

(56) Entgegenhaltungen:
- EP-A- 0 138 006
- EP-A- 0 234 437
- EP-A- 0 399 279
- EP-A- 0 409 066
- EP-A- 0 564 932
- MOLECULAR CRYSTALS AND LIQUID CRYSTALS, Bd.203, Juli 1991 Seiten 113 - 126 I.HEYNDERICKX ET AL. 'the use of cholesterically-ordered polymer networks in practical applications'
- LIQUID CRYSTALS, Bd.13, Nr.4, April 1991, LONDON GB Seiten 551 - 559 K.PRAEFKE ET AL. 'bis-1,3-diol type liquid crystals from pentaerythritol'

## Beschreibung

Wie für formanisotrope Moleküle bekannt, können beim Erwärmen flüssigkristalline Phasen, sogenannte Mesophasen, auftreten. Die einzelnen Phasen unterscheiden sich durch die räumliche Anordnung der Molekülschwerpunkte einerseits sowie durch die Molekülanordnung hinsichtlich der Längsachsen andererseits (G.W. Gray, P. A. Winsor, Liquid Crystals and Plastic Crystals, Ellis Horwood Limited, Chichester 1974). Die nematisch-flüssigkristalline Phase zeichnet sich dadurch aus, daß lediglich eine Orientierungsfernordnung durch Parallellagerung der Moleküllängsachsen existiert. Unter der Voraussetzung, daß die die nematische Phase aufbauenden Moleküle chiral sind, entsteht eine sogenannte cholesterische Phase, bei der die Längsachsen der Moleküle eine zu ihnen senkrechte, helixartige Überstruktur ausbilden (H. Baessler, Festkörperprobleme XI, 1971). Der chirale Molekülteil kann im flüssigkristallinen Molekül selbst enthalten sein oder aber als Dotierstoff zur nematischen Phase gegeben werden. Durch Dotierung erzeugte Phasen werden als induziert cholesterische Phasen bezeichnet. Dieses Phänomen wurde zuerst an Cholesterolderivaten untersucht (H. Baessler, M.M. Labes, J. Chem. Phys. 52 (1970) 631; H. Beassler, T.M. Laronge, M.M. Labes. J. Chem. Phys. 51 (1969) 3213; H. Finkelmann, H. Stegemeyer, Z. Naturforschg. 28a (1973) 799). Später wurde die Induzierung cholesterischer Phasen auch durch Zusatz anderer chiraler Substanzen möglich, die selbst nicht flüssigkristallin sind (H. Stegemeyer, K.J. Mainusch, Naturwiss. 58 (1971) 599; H. Finkelmann, H. Stegemeyer, Ber. Bunsenges. Phys. Chem. 78 (1974) 869).

Die cholesterische Phase hat bemerkenswerte optische Eigenschaften: eine hohe optische Rotation sowie einen ausgeprägten Circulardichroismus, der durch Selektivreflexion von circularpolarisiertem Licht innerhalb der cholesterischen Schicht entsteht. Die je nach Blickwinkel zu beobachtenden unterschiedlichen Farben sind abhängig von der Ganghöhe der helicalen Überstruktur, die ihrerseits vom Verdrillungsvermögen der chiralen Komponente abhängt. Dabei kann insbesondere durch Änderung der Konzentration eines chiralen Dotierstoffs die Ganghöhe und damit der Wellenlängenbereich des selektiv-reflektierten Lichts einer cholesterischen Schicht variiert werden (J.E. Adams, W.E.L. Haas, Mol. Cryst. Liq. Cryst. 16 (1972) 33). Solche cholesterischen Systeme bieten für eine praktische Anwendung interessante Möglichkeiten. So kann durch Einbau chiraler Molekülteile in mesogene Acrylsäureester nach Orientierung in der cholesterischen Phase und Photovernetzung ein stabiles, farbiges Netzwerk hergestellt werden, dessen Konzentration an chiraler Komponente aber nicht verändert werden kann (G. Galli, M. Laus, A. Angeloni, Makromol. Chem. 187 (1986) 289). Ferner kann durch Zumischen von nichtvernetzbaren chiralen Verbindungen zu nematischen Acrylsäureestern nach Photovernetzung ein farbiges Polymer hergestellt werden (I. Heynderickx, D.J. Broer, Mol. Cryst. Liq. Cryst. 203 (1991) 113), das jedoch noch flüchtige Bestandteile enthält, die für eine Anwendung prohibitiv sind.

Aufgabe der vorliegenden Erfindung war daher die Synthese neuer chiraler Verbindungen, die zum einen ein hohes Verdrillungsvermögen aufweisen und zum anderen in beliebiger Konzentration stabil in die cholesterische Phase eingebaut werden können, ohne daß sie aus der Phase herausdiffundieren oder kristallisieren können.

Die Erfindung betrifft daher polymerisierbare chirale Verbindungen, die mindestens eine zwei- oder mehrfach gebundene chirale Gruppe, welche sich von Zuckern, bi- oder polyfunktionellen Verbindungen aus der Biphenyl- oder Binaphthylreihe, von optisch aktiven Glykolen, Dialkoholen oder Aminosäuren ableitet, mindestens eine polymerisierbare Gruppe, mindestens einen üblichen Spacer und mindestens eine übliche mesogene Gruppe enthalten.

Weiter betrifft die Erfindung die Verwendung der erfindungsgemäßen Verbindungen in elektrooptischen Anzeigen oder als chiraler Dotierstoff für nematische oder cholesterische Flüssigkristalle zur Erzeugung farbig reflektierender Schichten und cholesterische Netzwerke, enthaltend die erfindungsgemäßen Verbindungen in vernetzter Form.

Als polymerisierbare Gruppen sind insbesondere Vinylreste zu nennen, die z.B. in Acrylverbindungen, Vinylethern oder Styrolderivaten enthalten sind. Daneben kommen auch Epoxide in Betracht.

Spacer und mesogene Gruppen sind die üblicherweise für diese Zwecke verwendeten Reste.

Die für die erfindungsgemäßen Verbindungen notwendigen Gruppen sind über Brückenglieder wie O, COO, OCO, CONH, NHCO, CON(R), N(R)CO oder auch eine direkte Bindung miteinander verknüpft.

Insbesondere betrifft die Erfindung Verbindungen der allgemeinen Formel I

(Z-Y-A-Y-M-Y-)ₙ X I,

in der - jeweils unabhängig voneinander - die Variablen die folgende Bedeutung haben
- A: ein Spacer, bestehend aus einer Kohlenstoffkette mit 2 bis 30 C-Atomen, welche durch O, S, NH oder NCH₃ unterbrochen sein kann und welche mit Fluor, Chlor, Brom, Cyan, Methyl oder Ethyl substituiert sein kann,
- M: eine Gruppe der Formel (T-Y¹)ᵣ-T,
- Y: eine direkte Bindung, O, S, COO, OCO, CON(R) oder N(R)CO,
- Z: eine polymerisierbare Gruppe 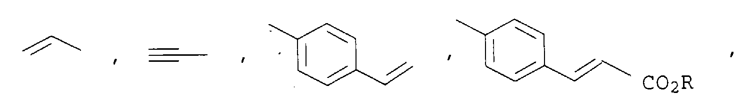 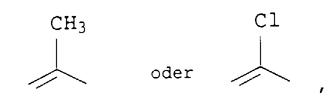
- n: eine der Zahlen 2 bis 6,
- X: eine chirale Gruppe, welche sich von Zuckern, bi- oder polyfunktionellen Verbindungen aus der Biphenyl- oder Binaphthylreihe, von optisch aktiven Glykolen, Dialkoholen oder Aminosäuren ableitet,
- R: C₁- bis C₄-Alkyl oder Wasserstoff,
- T: Cycloalkylen, ein Aromat oder Heteroaromat,
- Y¹: O, COO, OCO, CH₂O, OCH₂, CH=N oder N=CH oder eine direkte Bindung und
- r: 0 bis 3.

Üblicherweise sind die Spacer über Ester- oder Ethergruppen oder eine direkte Bindung mit X verknüpft. Die Spacer enthalten vorzugsweise 2 bis 12 C-Atome.

Insbesondere betrifft die Erfindung weiter Verbindungen der Formel I'

(Z-Y-A-Y-M-Y-)ₙ X I',

in der - jeweils unabhängig voneinander - die Variablen die folgende Bedeutung haben
- A: ein Spacer (CH₂)ₚ,
- p: 1 bis 12,
- M: eine Gruppe der Formel (T-Y¹)ᵣ-T,
- Y: eine direkte Bindung, O, S, COO, OCO, CON(R) oder N(R)CO,
- Z: eine polymerisierbare Gruppe 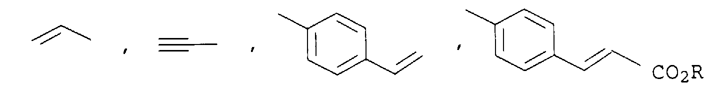 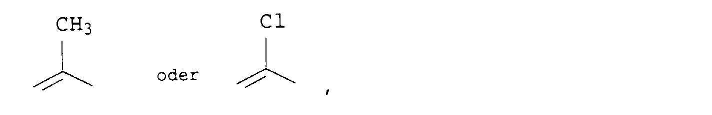
- n: eine der Zahlen 2 bis 6,
- X: eine chirale Gruppe, welche sich von Zuckern, bi- oder polyfunktionellen Verbindungen aus der Biphenyl- oder Binaphthylreihe, von optisch aktiven Glykolen, Dialkoholen oder Aminosäuren ableitet,
- R: C₁- bis C₄-Alkyl oder Wasserstoff,
- T: Cycloalkylen, ein Aromat oder Heteroaromat,
- Y¹: O, COO, OCO, CH₂O, OCH₂, CH=N oder N=CH oder eine direkte Bindung und
- r: 0 bis 3.

Repräsentative Spacer sind beispielsweise:
(CH₂)ₚ, (CH₂CH₂O)ₘCH₂CH₂, CH₂CH₂SCH₂CH₂, CH₂CH₂NHCH₂CH₂, 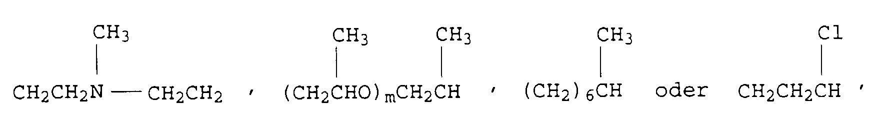 wobei
- m: 1 bis 3 und
- p: 1 bis 12 sind.

Vorzugsweise ist in den Verbindungen der Formeln I und I'r gleich 0 oder 1.

Die Reste T sind nicht aromatisch oder aromatisch carbocyclische oder heterocyclische, gegebenenfalls durch Fluor, Chlor, Brom, Cyan, Hydroxy oder Nitro substituierte Ringsysteme, die z.B. folgenden Grundstrukturen entsprechen:

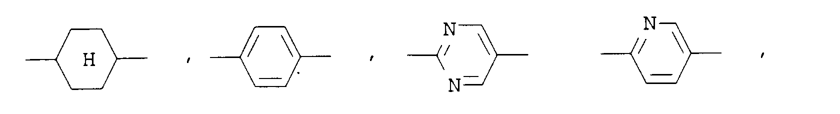

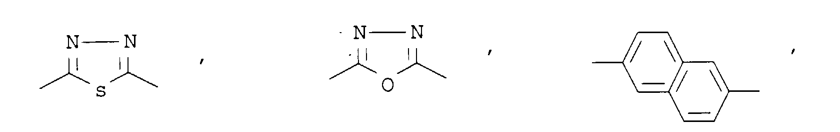

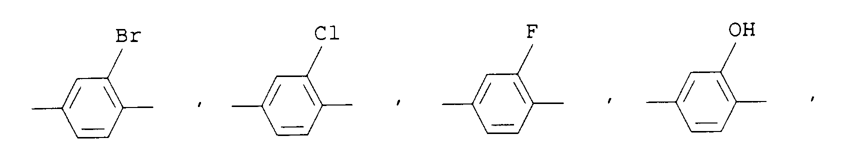

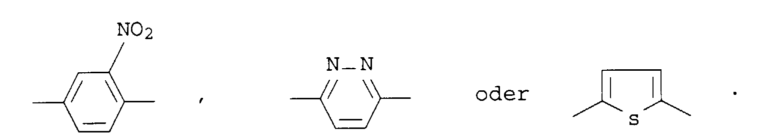

Besonders bevorzugt sind als mesogene Gruppen M z.B.: 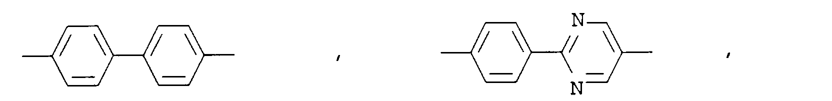 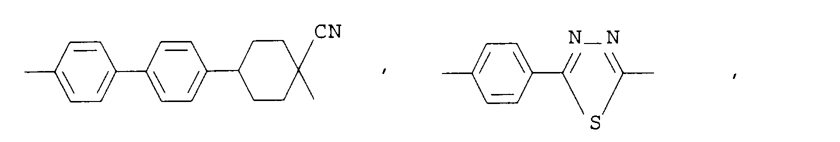 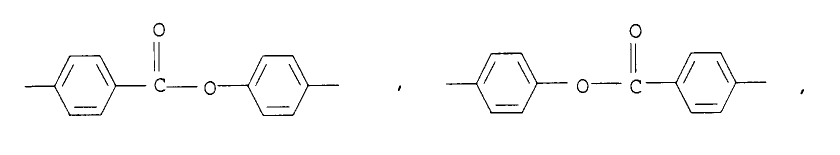 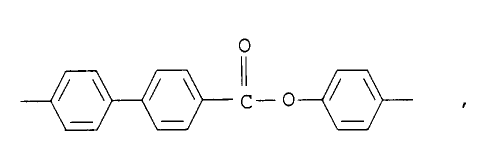 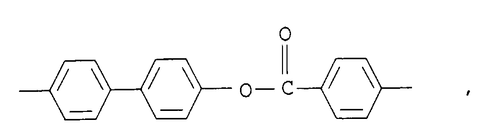 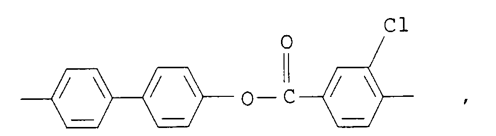 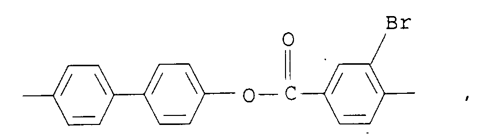 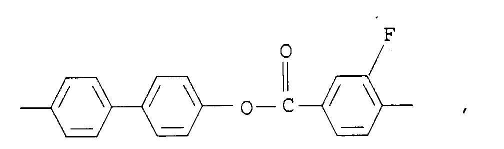 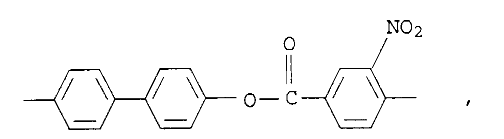 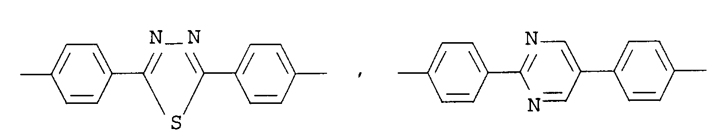 oder 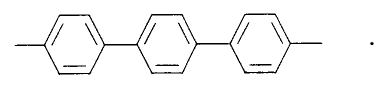 n in Formel I und I'ist vorzugsweise 2 oder 3 und insbesondere 2.

Einzelne Gruppen X sind z.B.: 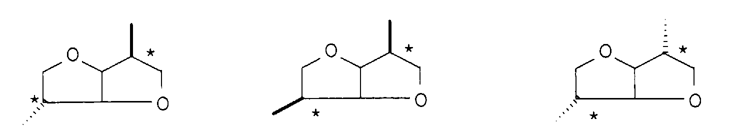 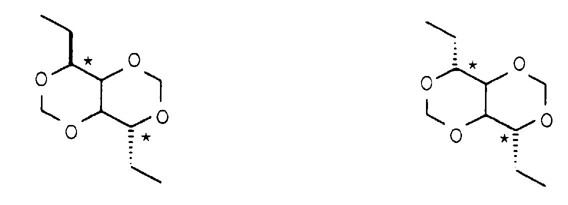 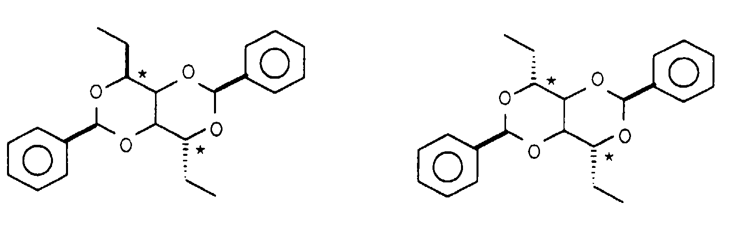 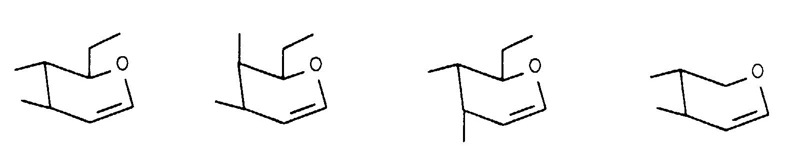 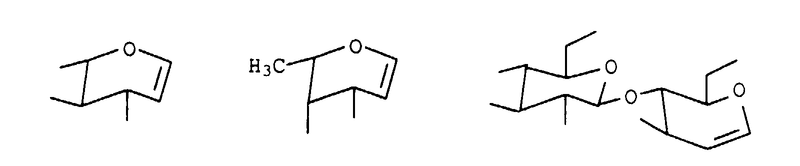 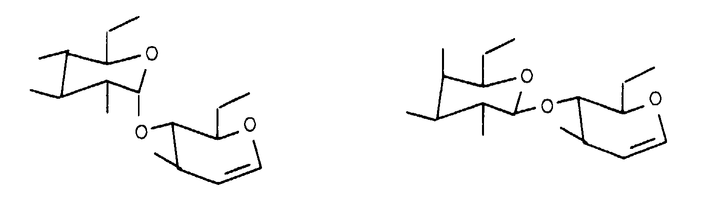 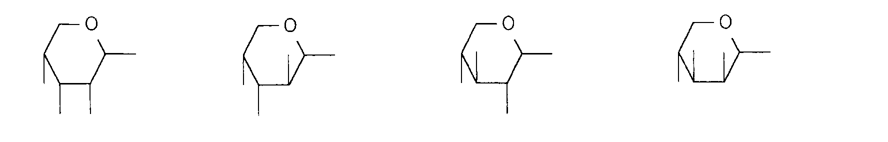 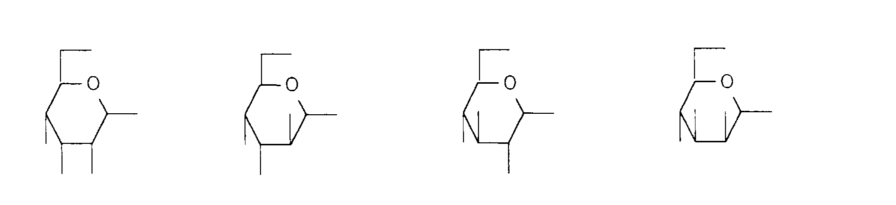 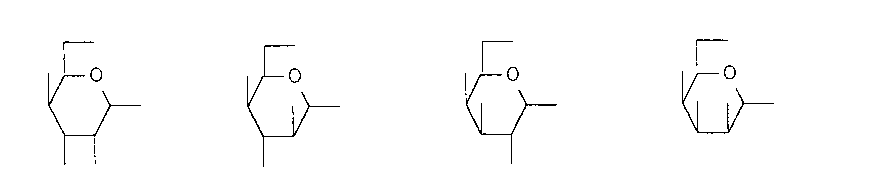 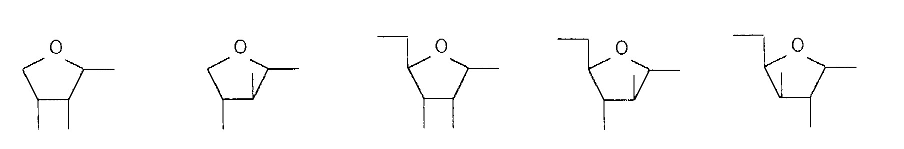 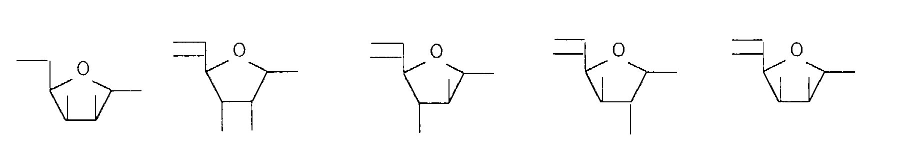 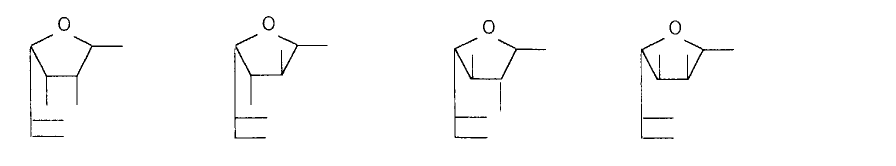 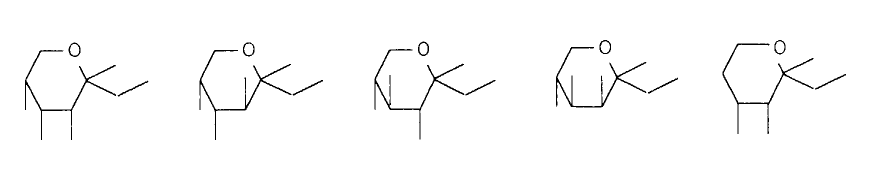 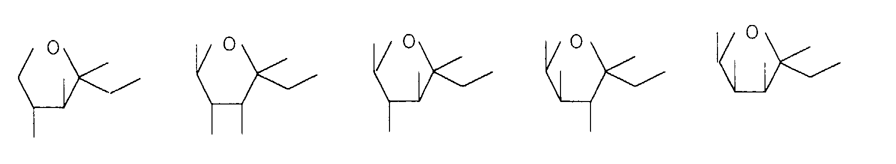 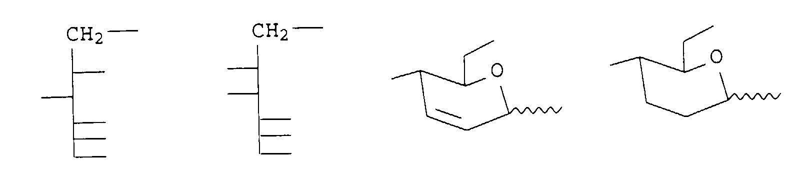 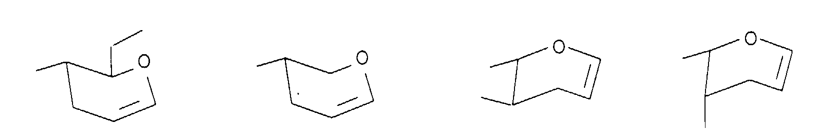 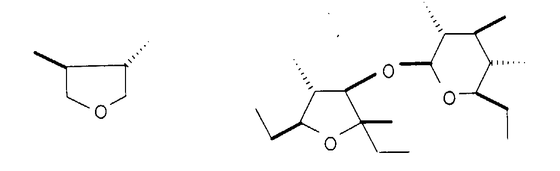 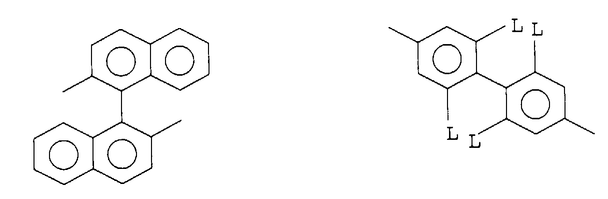 wobei
- L: C₁- bis C₄-Alkyl, C₁-C₄-Alkoxy, Halogen, COOR, OCOR, CONHR oder NHCOR ist und R die angegebene Bedeutung hat.

Besonders bevorzugt sind

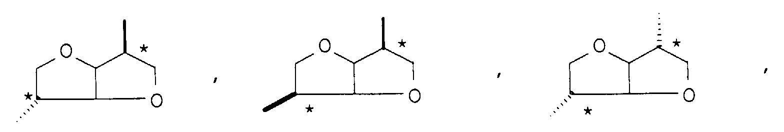

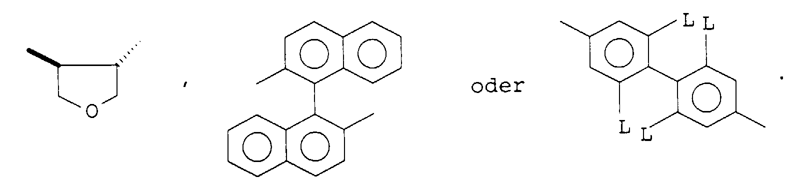

Optisch aktive Glykole oder Derivate davon entsprechen z.B. der Formel 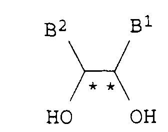 in der die Reste
- B¹ und B²: unabhängig voneinander C₁- bis C₄-Alkyl, das noch durch Hydroxy substituiert und durch -O- unterbrochen sein kann, Phenyl oder gegebenenfalls substituiertes Carboxyl und einer der Reste auch Wasserstoff sind, wobei bei gleichen Resten B¹ und B² die Konfiguration R,S ausgeschlossen ist.

Einzelne derartige Reste B¹ und B² sind z.B.
CO₂CH₃, CO₂CH₂CH₃, CO₂(CH₂)₂CH₃, CO₂(CH₂)₃CH₃, CO₂CH(CH₃)₂,
CO₂C(CH₃)₃ oder

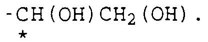

Weiterhin sind auch spezielle bifunktionelle chirale Gruppen geeignet, die folgende Strukturen aufweisen:

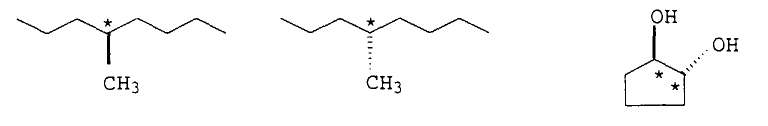

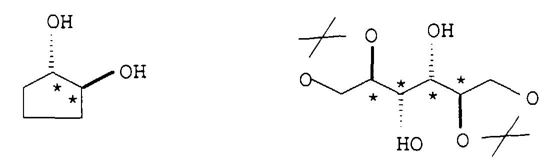

Bevorzugte Reste Z sind: 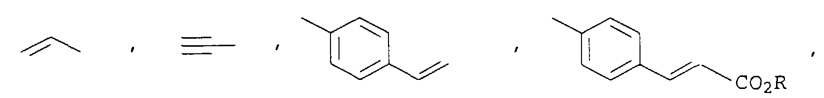 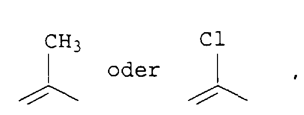 wobei R die angegebene Bedeutung hat.

Die erfindungsgemäßen Einheiten Z-Y-A-Y-M-Y, in denen Z, Y, A und M die oben angegebene Bedeutung haben, sind durch allgemein bekannte Syntheseverfahren, wie sie beispielsweise in der Schrift DE-A 39 17 196 beschrieben sind, zugänglich.

Die den chiralen Molekülteilen zugrundeliegenden Verbindungen können käuflich erworben werden und sind somit verfügbar.

Die erfindungsgemäßen Verbindungen eignen sich insbesondere zur Verwendung in elektro-optischen Anzeigeelementen oder als chiraler Dotierstoff für nematische oder cholesterische Flüssigkristalle zur Erzeugung farbig reflektierender Schichten.

### Beispiel 1

2,5-Bis-[4'-(2-acryloyloxy-ethoxy)-biphenyl-4-carbonyloyl]-1,4;3,6-dianhydro-D-sorbitol 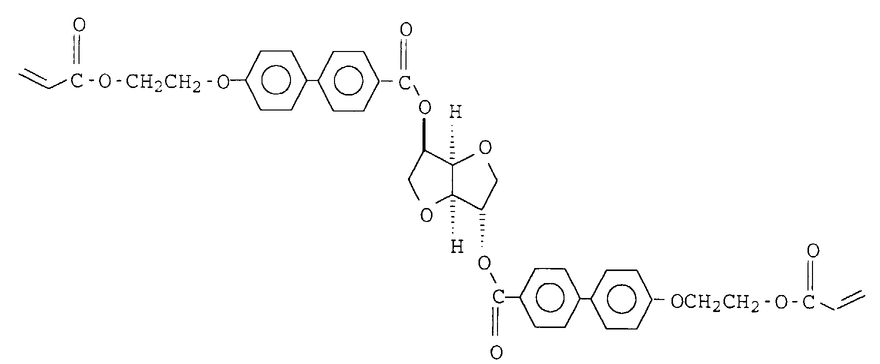
a 4'-Hydroxyethoxy-biphenyl-4-carbonsäureethylester 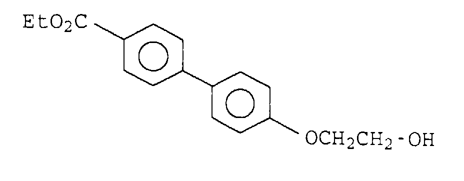 72,6 g (0,3 mol) 4'-Hydroxy-biphenyl-4-carbonsäureethylester werden in 225 ml abs. Dimethylformamid gelöst und mit 45,5 g (0,33 mol) Kaliumcarbonat sowie 3,0 g Kaliumiodid versetzt. Dann werden 26,57 g (0,33 mol) 2-Chlorethanol zugegeben und das Gemisch wird für 5 h auf 100°C erhitzt. Nach dem Rühren über Nacht bei Raumtemperatur werden weitere 22,77 g (0,17 mol) Kaliumcarbonat und 13,3 g (0,17 mol) 2-Chlorethanol zugegeben. Es wird weitere 15 h auf 100°C erhitzt, dann abgekühlt und auf Wasser gefällt. Der feste Rückstand wird mit Wasser neutral gewaschen und getrocknet. Das feuchte Produkt kann sofort weiter umgesetzt werden.
   Ausbeute: 123 g feuchtes Produkt, Fp. (Reinsubstanz) 128-129°C.
b 4'-Hydroxyethoxy-biphenyl-4-carbonsäure 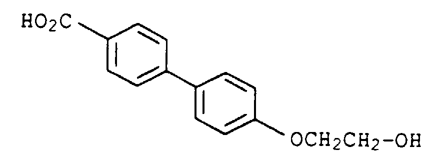 123 g (ca. 0,3 mol) wasserfeuchter 4'-Hydroxyethoxy-biphenyl-4-carbonsäureethylester werden in 258 ml Ethanol gelöst und mit 67,22 g (0,6 mol) 50 %iger KOH-Lösung versetzt. Nach einstündigem Erhitzen auf Rückfluß wird abgekühlt, der Rückstand abfiltriert, mit Ethanol gewaschen und trockengesaugt. Das Rohprodukt wird in Wasser aufgerührt und mit verd. Salzsäure angesäuert. Nach mehrstündigem Rühren wird das Produkt abgesaugt, mit Wasser neutral gewaschen und getrocknet.
   Ausbeute: 68,0 g ≙ 88 %, Fp. 155°C.
c 4'-(2-Acryloyloxy-ethoxy)-biphenyl-4-carbonsäure 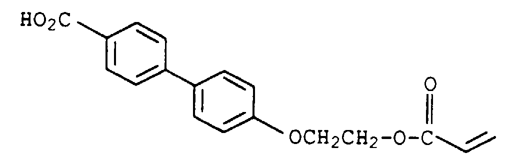 38,7 g (0,15 mol) 4'-Hydroxyethoxy-biphenyl-4-carbonsäure werden in 220 ml 1,1,1-Trichlorethan gelöst und mit 54,0 g (0,75 mol) frisch destillierter Acrylsäure und 0,5 g Hydrochinon versetzt. Nach Zugabe von 10,0 g p-Toluolsulfonsäure wird 4 h am Wasserabscheider zum Rückfluß erhitzt. Danach werden nochmals 54,0 g (0,75 mol) destillierte Acrylsäure zugefügt und weitere 3,5 h erhitzt, bis alles gelöst ist. Nach dem Abkühlen wird abgesaugt, mit 1,1,1-Trichlorethan gewaschen und anschließend mit t-Butylmethylether und Wasser verrührt. Der feste Rückstand wird abgesaugt, mit t-Butylmethylether gewaschen, getrocknet und aus 1,4 l Essigsäureethylester umkristallisiert.
   Ausbeute: 19,0 g ≙ 41 %.
d Acrylsäure-2-(4'-chlorcarbonyl-biphenyl-4-yloxy)-ethylester 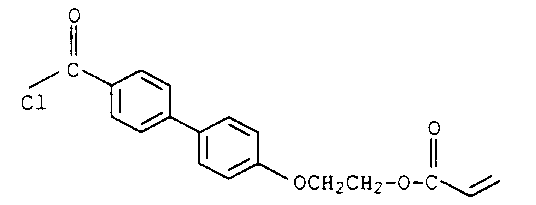 9,36 g (0,03 mol) 4'-(2-Acryloyloxy-ethoxy)-biphenyl-4-carbonsäure werden in 25 ml Oxalylchlorid gegeben und mit einem Tropfen Dimethylformamid versetzt. Als Radikalinhibitor wird eine Spatelspitze 2,6-Di-t-butyl-methylphenol zugesetzt, dann das Reaktionsgemisch während 35 min auf 40 - 50°C erhitzt. Anschließend wird das überschüssige Oxalylchlorid am Wasserstrahlvakuum abdestilliert, der verbliebene ölige Rückstand über Nacht am Ölpumpenvakuum getrocknet.
   Das Produkt kann direkt weiterverarbeitet werden.
   Ausbeute: 10,1 g ≙ 99 %.
e 2, 5-Bis- [4'-(2-acryloyloxy-ethoxy) -biphenyl-4-carbonyloyl]-1,4;3,6-dianhydro-D-sorbitol 1,99 g (0,014 mol) 1,4;3,6-Dianhydro-D-sorbitol werden in 50 ml abs. Dichlormethan gelöst, dann mit 2,37 g (0,03 mol) abs. Pyridin und einer Spatelspitze 2,6-Di-t-butyl-methylphenol und anschließend bei 0 - 5°C tropfenweise mit 9,93 g (0,03 mol) Acrylsäure-2-(4'-chlorcarbonyl-biphenyl-4-yloxy)-ethylester gelöst in 20 ml abs. Dichlormethan versetzt. Das Gemisch wird unter langsamem Erwärmen über Nacht gerührt, dann mit Wasser und wenig verd. Salzsäure versetzt und mehrmals ausgeethert. Die vereinigten org. Phasen werden mit Wasser gewaschen und nach dem Trocknen mit Na₂SO₄ vom Lösungsmittel befreit. Das Produkt wird durch Säulenchromatographie (Kieselgel, Laufmittel: Toluol/Essigsäureethylester 8:2) gereinigt.
   Ausbeute: 0,91 g ≙ 9 %, Fp. > 175°C.
   ¹H-NMR (200 MHz, CDCl₃):
   δ = 4,11 (d, J = 6,3 Hz, 2H, -CH₂-, Ring-H), 4,15 (m, 2H, -CH₂-, Ring-H), 4,25 (t, J = 6 Hz, 4H, -CH₂-OAr), 4,55 (t, J = 6 Hz, 4H, -CH₂-OCOR), 4,75 (d, J = 6 Hz, 1H, Brücken-H). 5,13 (t, J = 6Hz, 1H, Brücken-H), 5,45 (q, J = 6 Hz, 1H, Ring-H), 5,55 (m, 1H, Ring-H), 5,88 (d, J = 10,6 Hz, 2H, olef. H), 6,20 (dd, J = 17 Hz, J' = 10,6 Hz, 2H, olef. H), 7,0 (d, J = 8,6 Hz, 4H, arom. H), 7,5-7,7 (m, 8H, arom. H), 8,06 (d, J = 8,6 Hz, 2H, arom. H), 8,13 (d, J = 8,6 Hz, 2H, arom. H).

### Beispiel 2

2,5-Bis-[4'-(2-acryloyloxy-ethoxy)-phenyl-4-carbonyloyl]-1,4;3,6-dianhydrid-D-sorbitol 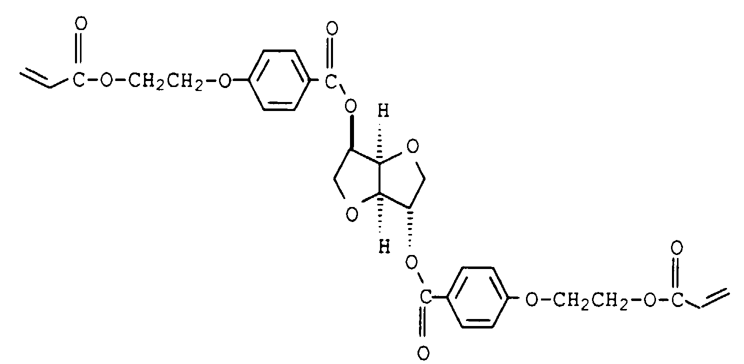 Die Verbindung wurde analog Beispiel 1 unter Verwendung von 4-Hydroxybenzoesäureethylester hergestellt.
Ausbeute: 1,24 g ≙ 16 %, Fp. > 156°C.
¹H-NMR (200 MHz, CDCl₃):
δ = 4,05 (d, J - 5,7 Hz, 2H, -CH₂-, Ring-H), 4,12 (m, 2H, -CH₂-, Ring-H), 4,24 (t, J = 6 Hz, 4H, -CH₂-OAr), 4,56 (t, J = 6 Hz, 4H, -CH₂-OCOR), 4,70 (d, J = 6 Hz, 1H, Brücken-H), 5,08 (t, J = 6Hz, 1H, Brücken-H), 5,40 (q, J = 6 Hz, 1H, Ring-H), 5,46 (m, 1H, Ring-H), 5,82 (d, J = 10,7 Hz, 2H, olef. H), 6,18 (dd, J = 17 Hz, J' = 10,7 Hz, 2H, olef. H), 6,48 (d, J = 17 Hz, 2H, olef. H), 6,95 (d, J = 8,3 Hz, 2H, arom. H), 7,0 (d, J = 8,3 Hz, 2H, arom. H), 7,95 (d, J = 8,3 Hz, 2H, arom. H), 8,05 (d, J = 8,3 Hz, 2H, arom. H).

### Beispiel 3

2,5-Bis-[4'-(2-acryloyloxy-ethoxy)-biphenyl-4-carbonyloyl]-1,4;3,6-dianhydro-D-mannitol 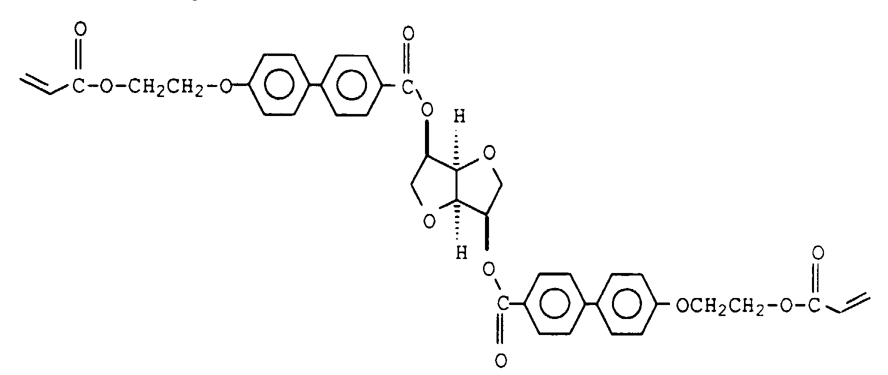 Die Verbindung wurde analog Beispiel 1 unter Verwendung von 1,4;3,6-Dianhydro-D-mannitol hergestellt.
Ausbeute: 1,18 g ≙ 12 %, Fp. > 195°C.
¹H-NMR (200 MHz, CDCl₃):
δ = 3,82 (dd, J = 6,3 Hz, J' = 3 Hz, 2H, -CH₂-, Ring-H), 3,88 (dd, J = 6,3 Hz, J' = 3 Hz, 2H, -CH₂-, Ring-H), 4,15 (t, J = 6 Hz, 4H, -CH₂-OAr), 4,4 (t, J = 6 Hz, 4H, -CH₂-OCOR), 4,8 (m, 2H, Brükken-H), 5,25 (m, 1H, Brücken-H), 5,35 (m, 1H, Ring-H), 5,85 (d, J = 10,4 Hz, 2H, olef. H), 6,15 (dd, J = 16 Hz, J' = 10,4 Hz, 2H, olef. H), 6,4 (d, J = 16 Hz, 2H, olef. H), 7,1 (d, J = 8,5 Hz, 4H, arom. H), 7,55 (d, J = 8,5 Hz, 4H, arom. H), 8,0 (d, J = 8,5 Hz, 4H, arom. H), 8,1 (d, J = 8,5 Hz, 4H, arom. H).

### Beispiel 4

2,5-Bis-[4'-(2-acryloyloxy-ethoxy)-biphenyl-4-carbonyloyl]-1,4;3,6-dianhydro-L-iditol 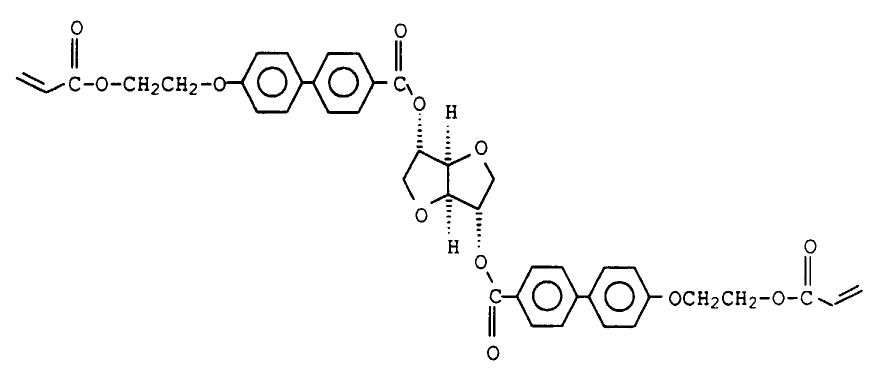 Die Verbindung wurde analog Beispiel 1 unter Verwendung von 1,4;3,6-Dianhydro-L-iditol hergestellt.
Ausbeute: 1,89 g ≙ 19 %, Fp. >195°C.
¹H-NMR (200 MHz, CDCl₃):
δ = 3,93 (dd, J = 11,0 Hz, J' = 3,0 Hz, 2H, -CH₂-, Ring-H), 3,98 (dd, J = 11,0 Hz, J' = 3 Hz, 2H, -CH₂-, Ring-H), 4,30 (t, J = 6 Hz, 4H, -CH₂-OAr), 4,50 (t, J = 6 Hz, 4H, -CH₂-OCOR), 5,35 (s, 2H, Brücken-H), 5,65 (dd, J = 11,0 Hz, J' = 3 Hz, 2H, Brücken-H), 5,90 (d, J = 10,7 Hz, 2H, olef.H), 6,20 (dd, J = 16,0 Hz, J' = 10,7 Hz, 2H, olef. H), 6,55 (d, J = 16 Hz, 2H, olef. H), 7,1 (d, J - 8,7 Hz, 4H, arom. H), 7,50 (d, J = 8,7 Hz, 4H, arom. H), 8,12 (d, J = 8,7 Hz, 4H, arom. H), 8,13 (d, J = 8,7 Hz, 4H, arom. H).

### Beispiel 5

2,5-Bis-[4'-(2-acryloyloxy-ethoxy)-biphenyl-4-carbonyloyl]-1,4;3,6-dianhydro-L-iditol 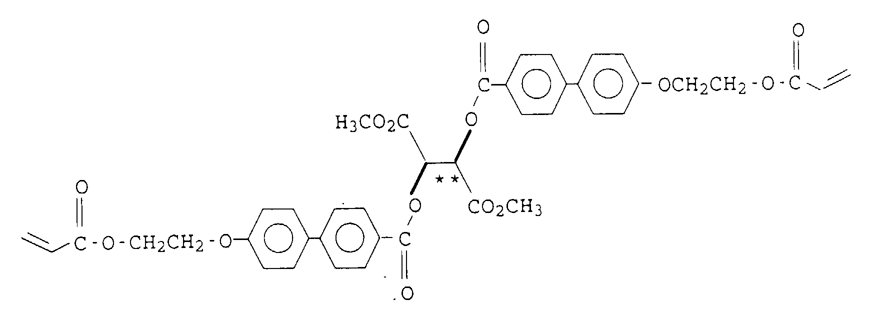 Die Verbindung wurde analog Beispiel 1 unter Verwendung von (R,R) -Weinsäurediethylester hergestellt.
Ausbeute: 2,22 g ≙ 20 %, Fp. 146°C.
¹H-NMR (200 MHz, CDCl₃):
δ = 1,28 (t, J = 6,9 Hz, 6H, Ester-CH₃), 4,15 (q, J = 6,9 Hz, 4H, Ester-CH₂), 4,3 (t, J = 6,0 Hz, 4H, -CH₂-O-Arom.), 4,55 (t, J = 6,0 Hz, 4H, -CH₂-O-COR), 5,88 (d, J = 11,3 Hz, 2H, olef. H), 6,04 (s, 2H, -CH(OR)(CO₂R)), 6,16 (dd, J = 17,3, J' = 11,3 Hz, 2H, olef. H), 6,48 (d, J = 17,3 Hz, 1H, olef.H), 7,05 (d, J = 8,6 Hz, 4H, arom. H), 7,57 (d, J = 8,6 Hz, 4H, arom. H), 7,68 (d, J = 7,6 Hz, 4H, arom. H), 8,16 (d, J = 7,6 Hz, 4H, arom. H).

Analog zu den beschriebenen Methoden lassen sich auch die in den folgenden Tabellen gekennzeichneten Verbindungen herstellen, die ähnliche physikalische Eigenschaften haben.

Beispiel 6 nicht vorhanden

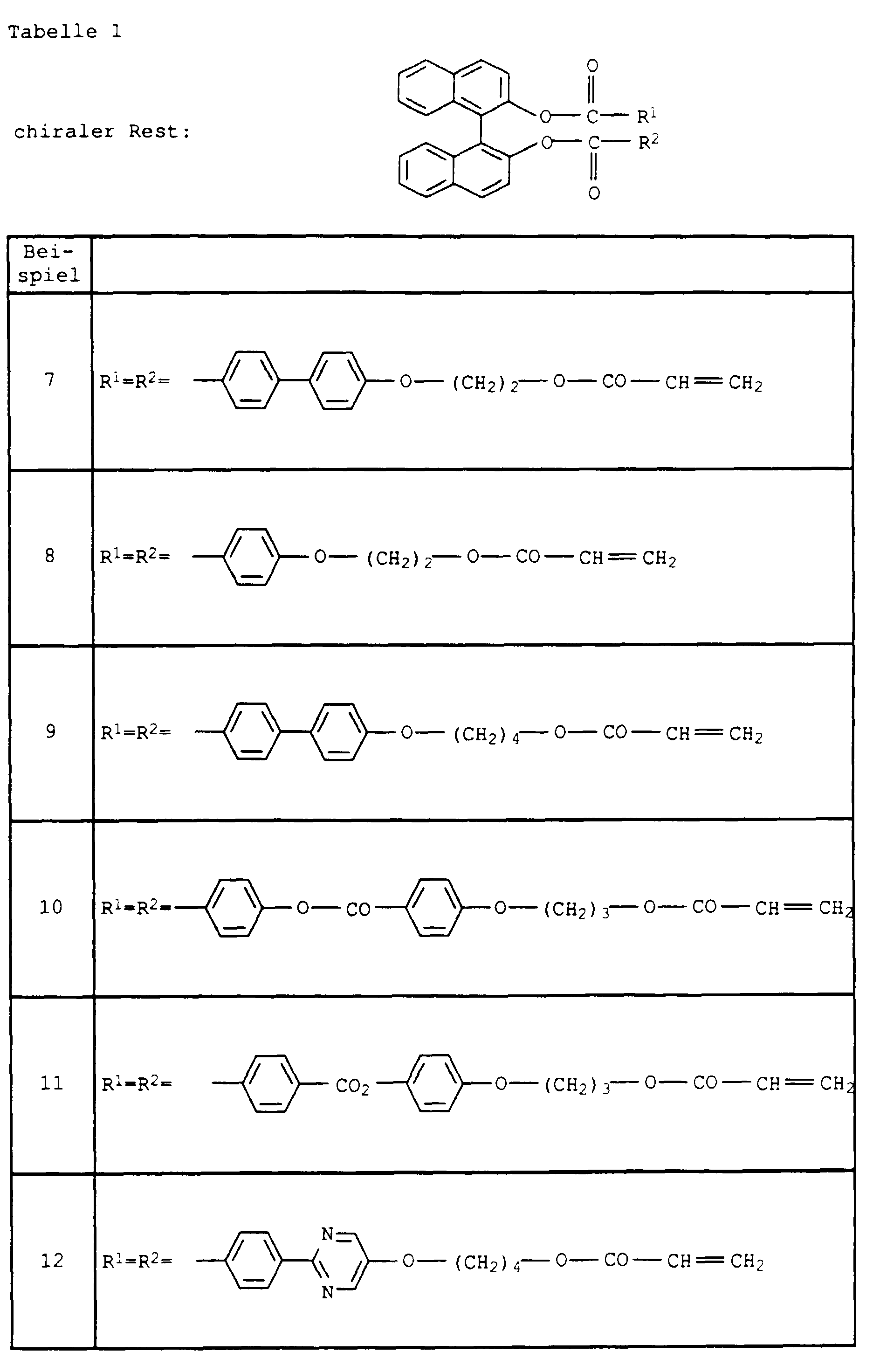

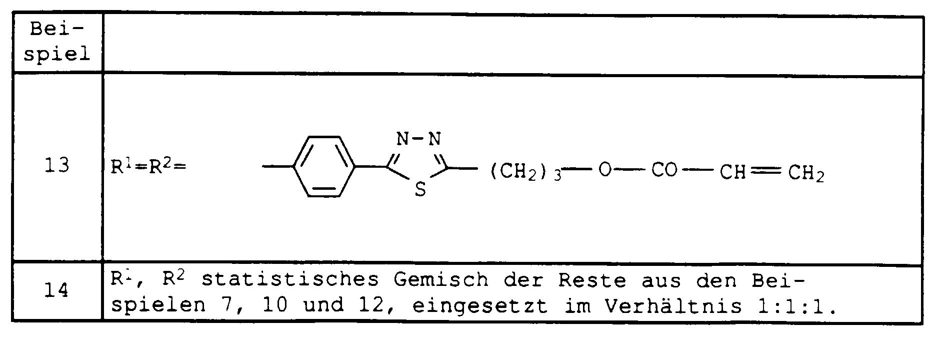

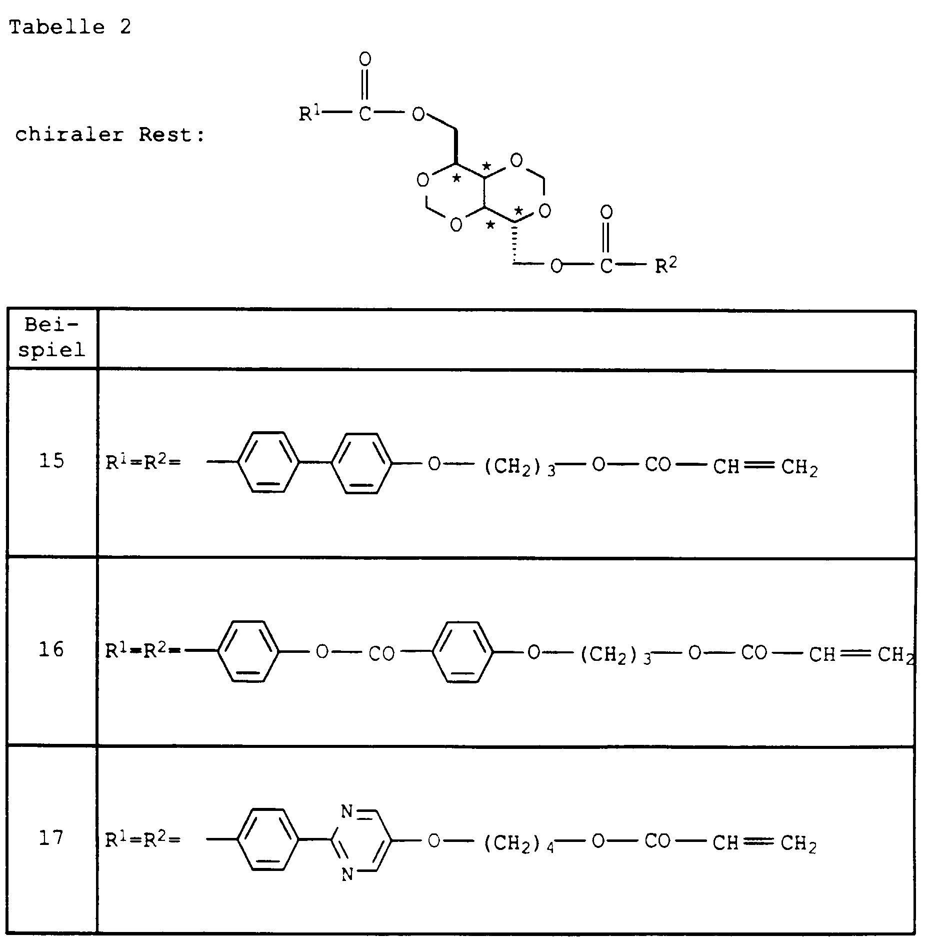

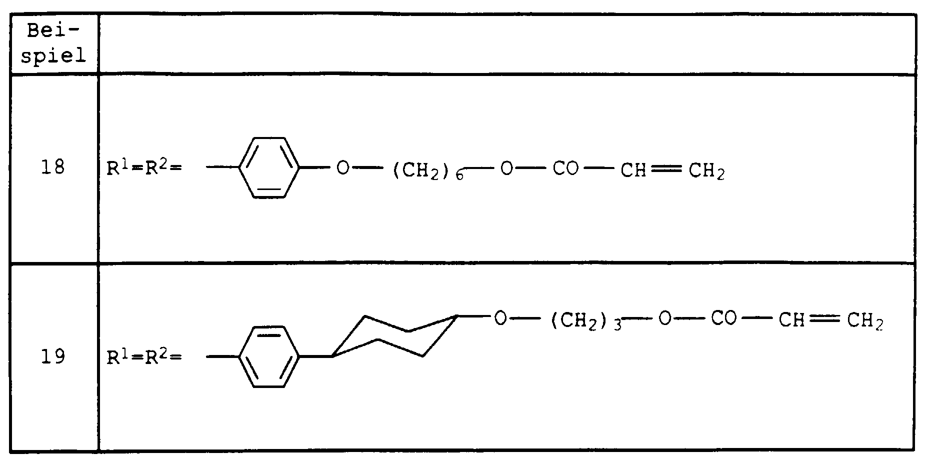

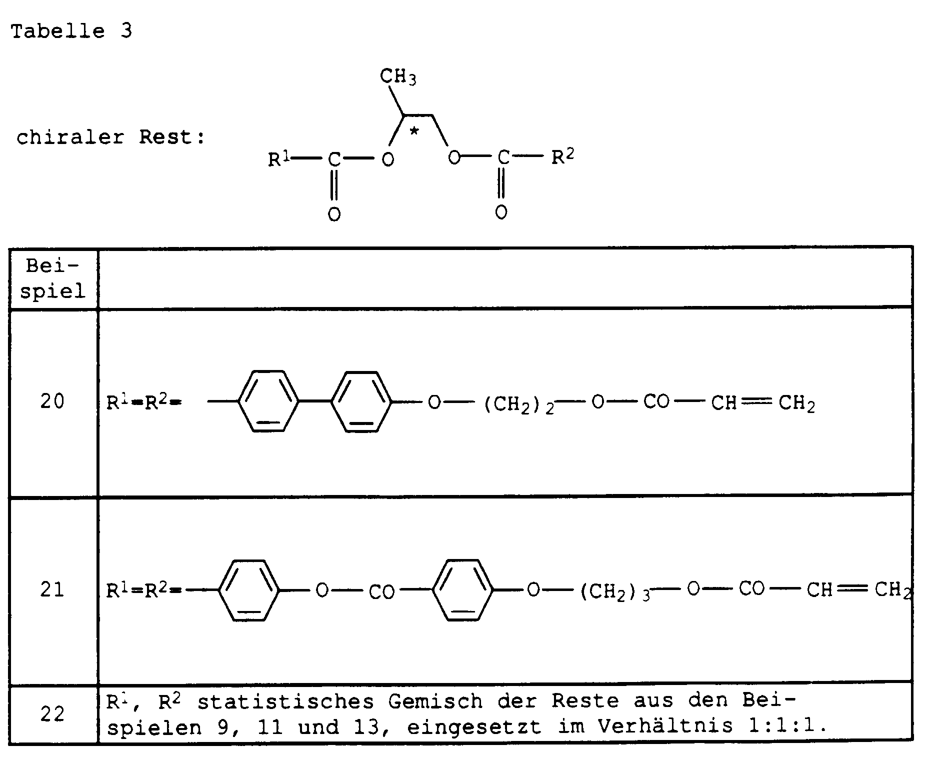

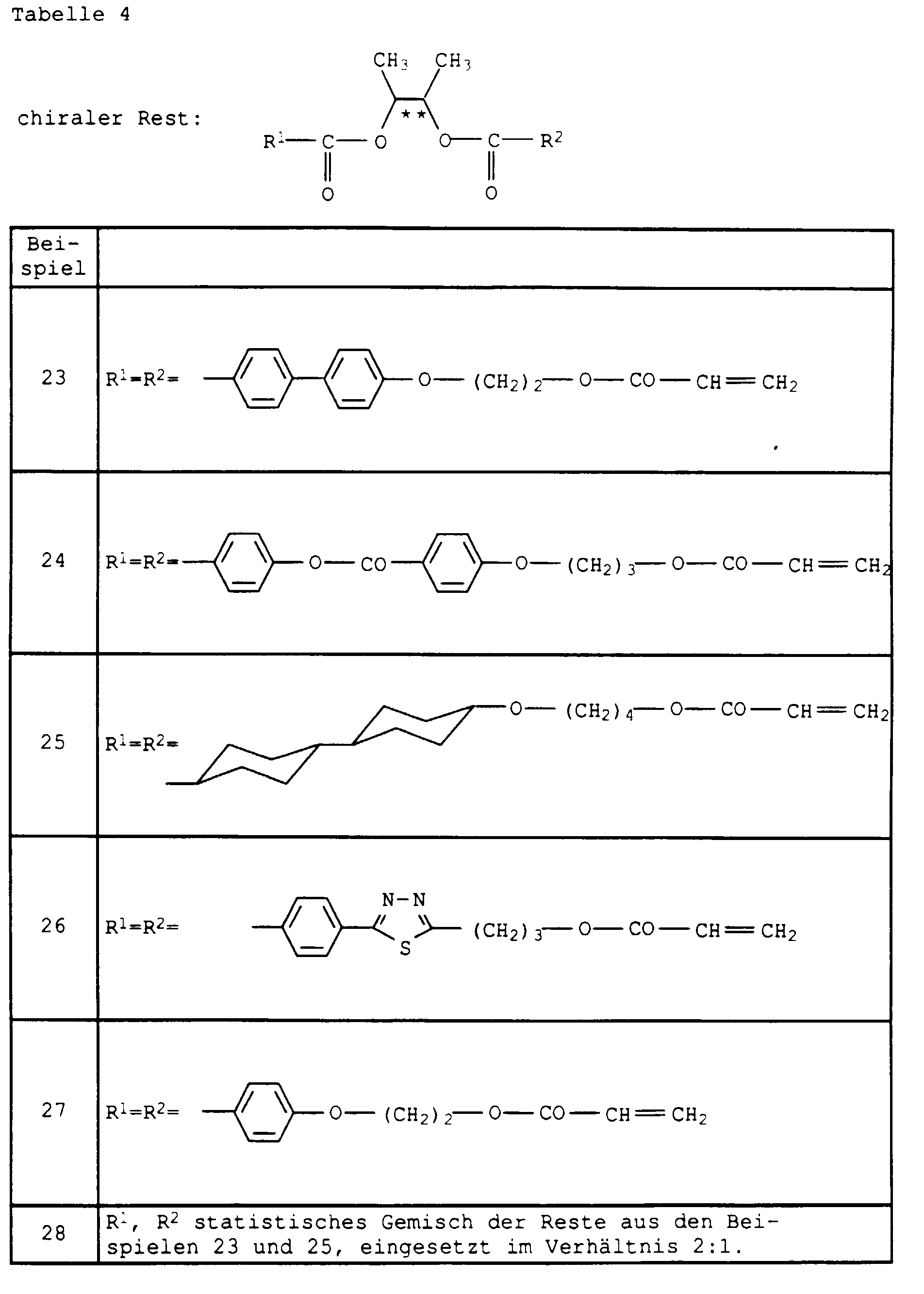

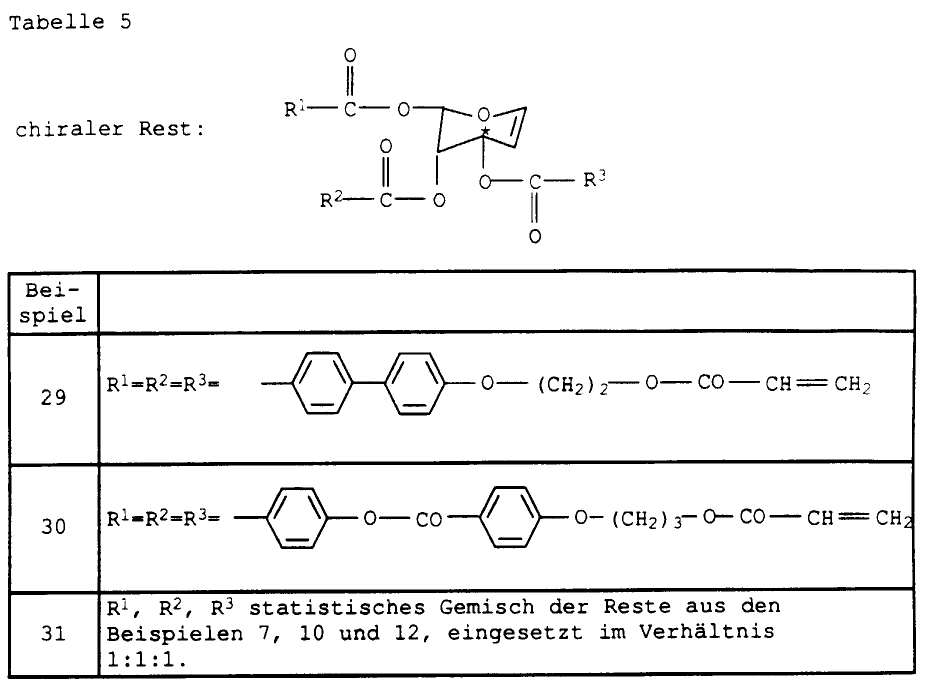

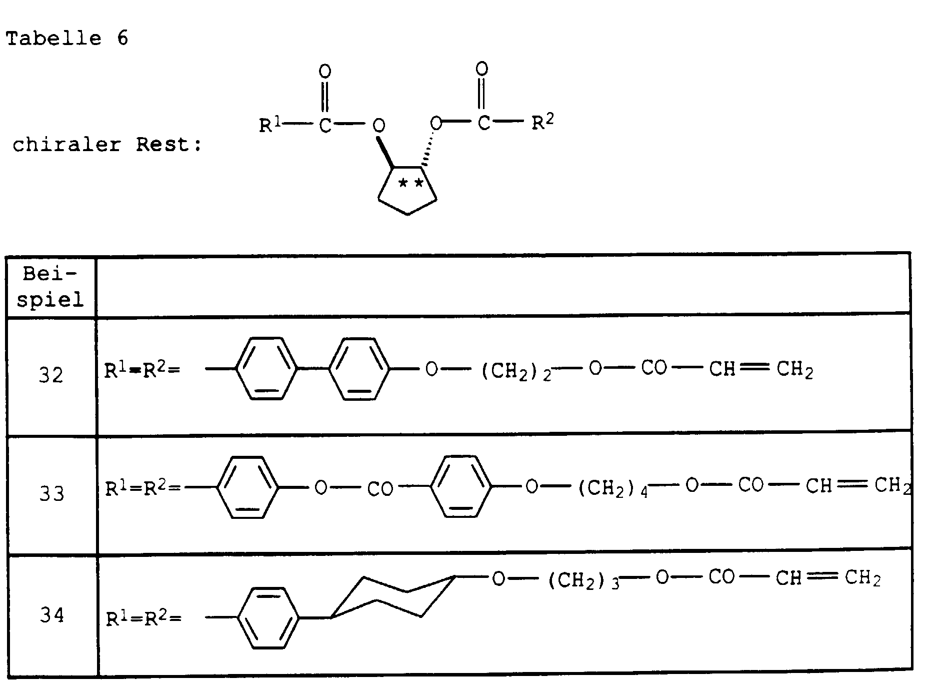

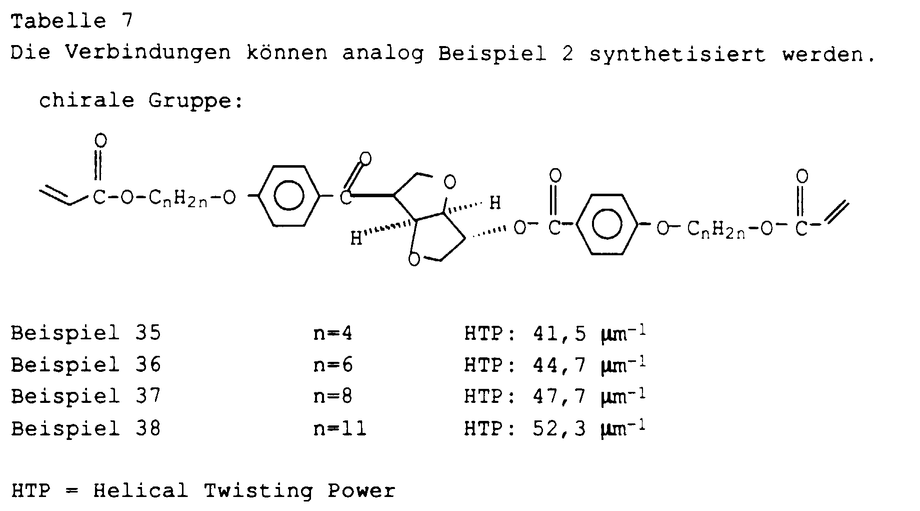

Entsprechend der Arbeitsweise von Beispiel 2 werden durch Umsetzung mit Methyl-4,6-benzyliden-α-D-glucopyranosid die Verbindungen der Beispiele 39 bis 42 erhalten.

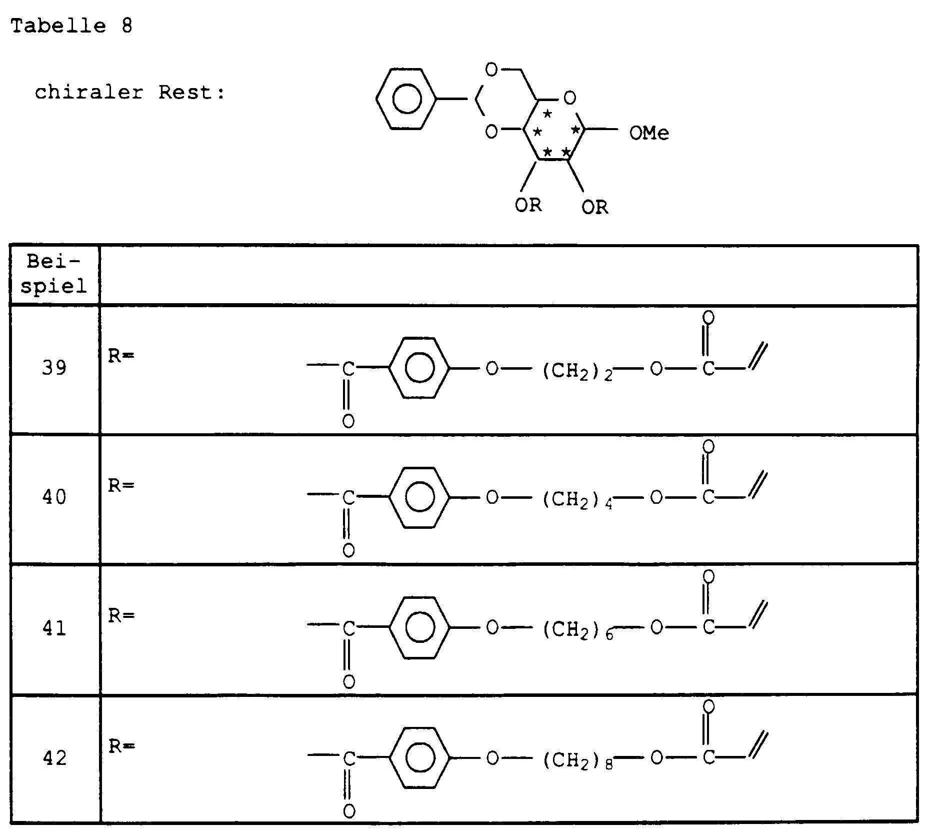

Analog Beispiel 2 werden durch Umsetzung des chiralen Restes mit 4'-(ω-Vinyloxy)alkylenoxyphenyl-4-carbonsäuren die Verbindungen der Beispiele 43 bis 47 erhalten

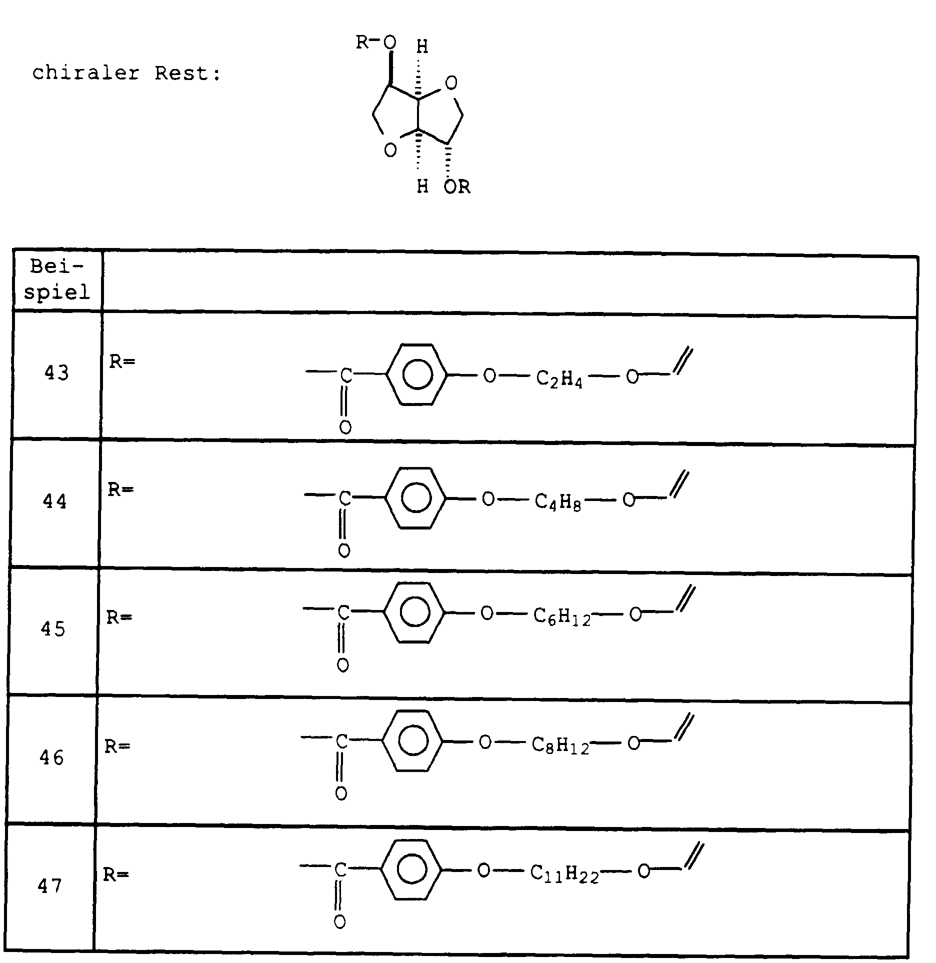

## Patentansprüche

1. Polymerisierbare chirale Verbindungen, die mindestens eine zwei- oder mehrfach gebundene chirale Gruppe, welche sich von Zuckern, bi- oder polyfunktionellen Verbindungen aus der Biphenyl- oder Binaphthylreihe, von optisch aktiven Glykolen, Dialkoholen oder Aminosäuren ableitet, mindestens eine polymerisierbare Gruppe, mindestens einen üblichen Spacer und mindestens eine übliche mesogene Gruppe enthalten.

2. Verbindungen der Formel
(Z-Y-A-Y-M-Y-)ₙ X I,
in der - jeweils unabhängig voneinander - die Variablen die folgende Bedeutung haben
A ein Spacer, bestehend aus einer Kohlenstoffkette mit 2 bis 30 C-Atomen, welche durch O, S, NH oder NCH₃ unterbrochen sein kann und welche mit Fluor, Chlor, Brom, Cyan, Methyl oder Ethyl substituiert sein kann,
M eine Gruppe der Formel (T-Y¹)ᵣ-T,
Y eine direkte Bindung, O, S, COO, OCO, CON(R) oder N(R)CO,
Z eine polymerisierbare Gruppe 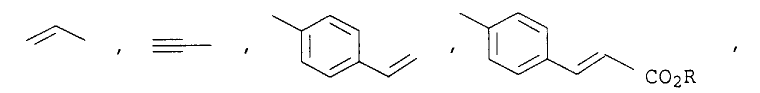 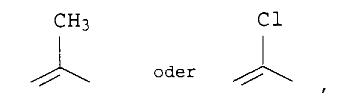
n eine der Zahlen 2 bis 6,
X eine chirale Gruppe, welche sich von Zuckern, bi- oder polyfunktionellen Verbindungen aus der Biphenyl- oder Binaphthylreihe, von optisch aktiven Glykolen, Dialkoholen oder Aminosäuren ableitet,
R C₁- bis C₄-Alkyl oder Wasserstoff,
T Cycloalkylen, ein Aromat oder Heteroaromat,
Y¹ O, COO, OCO, CH₂O, OCH₂, CH=N oder N=CH oder eine direkte Bindung und
r 0 bis 3.

3. Verbindungen der Formel
(Z-Y-A-Y-M-Y-)ₙ X I',
in der - jeweils unabhängig voneinander - die Variablen die folgende Bedeutung haben
A ein Spacer (CH₂)ₚ,
p 1 bis 12,
M eine Gruppe der Formel (T-Y¹)ᵣ-T,
Y eine direkte Bindung, O, S, COO, OCO, CON(R) oder N(R)CO,
Z eine polymerisierbare Gruppe 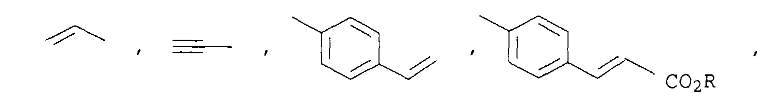 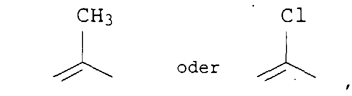
n eine der Zahlen 2 bis 6,
X eine chirale Gruppe, welche sich von Zuckern, bi- oder polyfunktionellen Verbindungen aus der Biphenyl- oder Binaphthylreihe, von optisch aktiven Glykolen, Dialkoholen oder Aminosäuren ableitet,
R C₁- bis C₄-Alkyl oder Wasserstoff,
T Cycloalkylen, ein Aromat oder Heteroaromat,
yl O, COO, OCO, CH₂O, OCH₂, CH=N oder N=CH oder eine direkte Bindung und
r 0 bis 3.

4. Verbindungen gemäß Anspruch 2 oder 3 mit r 0 oder 1.

5. Verbindungen gemäß Anspruch 1, in welchen die chirale Gruppe zweifach gebunden ist, oder gemäß Anspruch 2 oder 3 mit n = 2.

6. Verbindungen gemäß Anspruch 5, in welchen die chirale Gruppe die folgenden Reste bedeutet 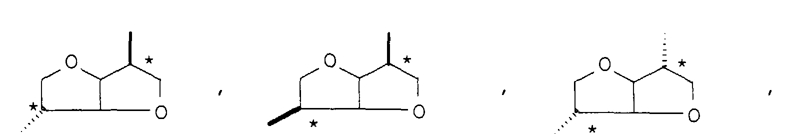 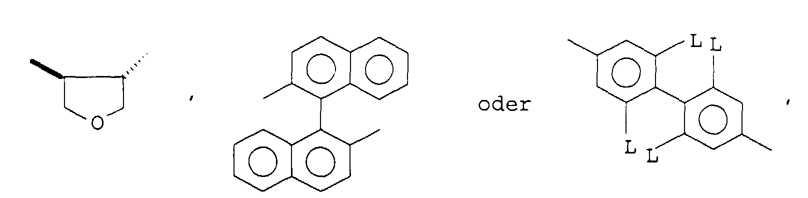 wobei
L C₁- bis C₄-Alkyl, C₁-C₄-Alkoxy, Halogen, COOR, OCOR, CONHR oder NHCOR ist und R die angegebene Bedeutung hat.

7. Verwendung der Verbindungen gemäß Anspruch 1 bis 6 in elektrooptischen Anzeigen oder als chiraler Dotierstoff für nematische oder cholesterische Flüssigkristalle zur Erzeugung farbig reflektierender Schichten.

8. Cholesterische Netzwerke, enthaltend Verbindungen gemäß Anspruch 1 bis 6 in vernetzter Form.

## Claims

1. A polymerizable chiral compound containing at least one divalent or polyvalent chiral group derived from a sugar, a bifunctional or polyfunctional compound from the biphenyl or binaphthyl series, from an optically active glycol, dialcohol or amino acid, at least one polymerizable group, at least one conventional spacer and at least one conventional mesogenic group.

2. A compound of the formula
(Z-Y-A-Y-M-Y-)ₙ X I,
where - in each case independently of one another - the variables have the following meanings:
A is a spacer consisting of a carbon chain having 2 to 30 carbon atoms, which may be interrupted by O, S, NH or NCH₃ and which may be substituted by fluorine, chlorine, bromine, cyano, methyl or ethyl,
M is a group of the formula (T-Y¹)ᵣ-T,
Y is a direct bond, O, S, COO, OCO, CON(R) or N(R)CO,
Z is a polymerizable group 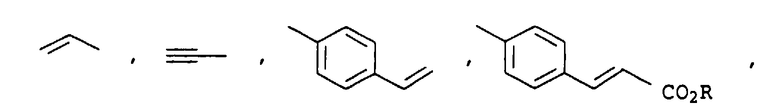 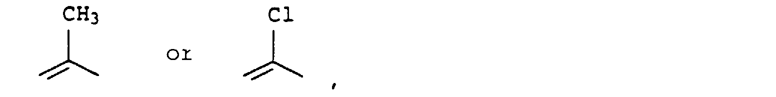
n is a number from 2 to 6,
X is a chiral group derived from a sugar, a bifunctional or polyfunctional compound from the biphenyl or binaphthyl series, from an optically active glycol, dialcohol or amino acid,
R is C₁- to C₄-alkyl or hydrogen,
T is cycloalkylene, an aromatic group or a heteroaromatic group,
Y¹ is O, COO, OCO, CH₂O, OCH₂, CH=N or N=CH or a direct bond, and
r is from 0 to 3.

3. A compound of the formula
(Z-Y-A-Y-M-Y-)ₙ X I',
where - in each case independently of one another - the variables have the following meanings:
A is a spacer (CH₂)ₚ,
p is from 1 to 12,
M is a group of the formula (T-Y¹)ᵣ-T,
Y is a direct bond, O, S, COO, OCO, CON(R) or N(R)CO,
Z is a polymerizable group 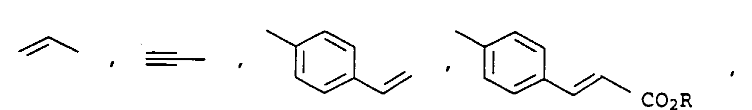 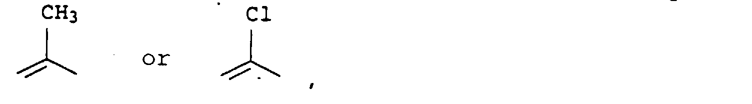
n is a number from 2 to 6,
X is a chiral group derived from a sugar, a bifunctional or polyfunctional compound from the biphenyl or binaphthyl series, from an optically active glycol, dialcohol or amino acid,
R is C₁- to C₄-alkyl or hydrogen,
T is cycloalkylene, an aromatic group or a heteroaromatic group,
Y¹ is O, COO, OCO, CH₂O, OCH₂, CH=N or N=CH or a direct bond, and
r is from 0 to 3.

4. A compound as claimed in claim 2 or 3, where r is 0 or 1.

5. A compound as claimed in claim 1, where the chiral group is divalent, or as claimed in claim 2 or 3, where n = 2.

6. A compound as claimed in claim 5 where the chiral group is one of the following radicals: 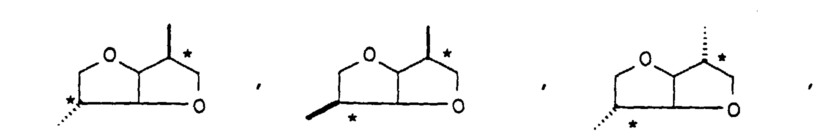 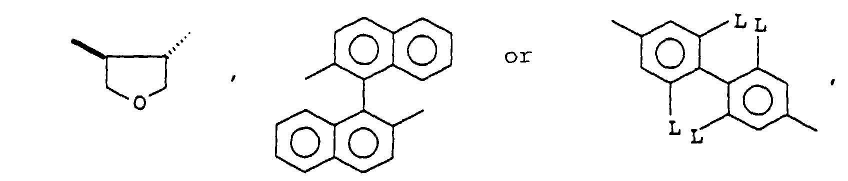 where
L is C₁- to C₄-alkyl, C₁-C₄-alkoxy, halogen, COOR, OCOR, CONHR or NHCOR, and R is as defined above.

7. The use of a compound as claimed in claims 1 to 6 in electro-optical displays or as a chiral dopant for nematic or cholesteric liquid crystals for generating layers which reflect in color.

8. A cholesteric network comprising compounds as claimed in claims 1 to 6 in crosslinked form.

## Revendications

1. Composés chiraux polymérisables, contenant au moins un groupement chiral lié deux ou plusieurs fois, dérivant de sucres, de composés bi- ou polyfonctionnels de la série des biphényles ou des binaphtyles, de glycols optiquement actifs, de dialcools ou d'aminoacides, au moins un groupement polymérisable, au moins un espaceur usuel et au moins un groupement mésogéne usuel.

2. Composés de formule
(Z-Y-A-Y-M-Y-)ₙ X I,
dans laquelle - à chaque fois indépendamment les unes des autres- les variables ont la signification suivante
A représente un espaceur, constitué d'une chaîne carbonée à 2-30 atomes de carbone, pouvant être interrompue par O, S, NH ou NCH₃, et pouvant être substituée par des atomes de fluor, de chlore, de brome, des groupements cyano, méthyle ou éthyle,
M représente un groupement de formule (T-Y¹)ᵣ-T,
Y représente une liaison directe, O, S, COO, OCO, CON(R) ou N(R)CO,
Z représente un groupement polymérisable 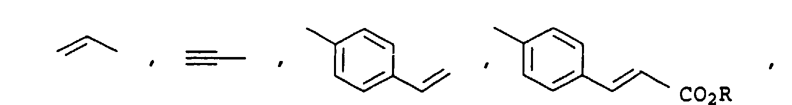 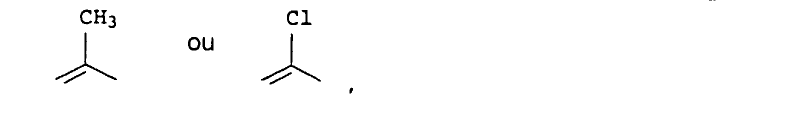
n représente un nombre de 2 à 6,
X représente un groupement chiral, dérivant de sucres, de composés bi- ou polyfonctionnels de la série des biphényles ou des binaphtyles, de glycols optiquement actifs, de dialcools ou d'aminoacides,
R représente un groupement alkyle en C₁-C₄ ou un atome d'hydrogène,
T représente un groupement cycloalkylène, un composé aromatique ou hétéroaromatique,
Y¹ représente O, COO, OCO, CH₂O, OCH₂, CH=N ou N=CH ou une liaison directe et
r vaut de 0 à 3.

3. Composés de formule
(Z-Y-A-Y-M-Y-)ₙ X I',
dans laquelle - à chaque fois indépendamment les unes des autres- les variables ont la signification suivante
A représente un espaceur (CH₂)ₚ,
p vaut de 1 à 12,
M représente un groupement de formule (T-Y¹)ᵣ-T,
Y représente une liaison directe, O, S, COO, OCO, CON(R) ou N(R)CO,
Z représente un groupement polymérisable 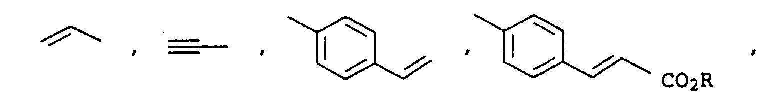 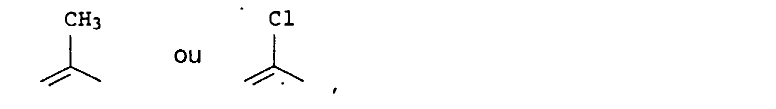
n représente un nombre de 2 à 6,
X représente un groupement chiral, dérivant de sucres, de composés bi- ou polyfonctionnels de la série des biphényles ou des binaphtyles, de glycols optiquement actifs, de dialcools ou d'aminoacides,
R représente un groupement alkyle en C₁-C₄ ou un atome d'hydrogène,
T représente un groupement cycloalkylène, un composé aromatique ou hétéroaromatique,
Y¹ représente O, COO, OCO, CH₂O, OCH₂, CH=N ou N=CH ou une liaison directe et
r vaut de 0 à 3.

4. Composés selon la revendication 2 ou 3 pour lesquels r vaut 0 ou 1.

5. Composés selon la revendication 1, pour lesquels le groupement chiral est lié deux fois, ou selon la revendication 2 ou 3 avec n=2.

6. Composés selon la revendication 5, pour lesquels le groupement chiral représente les restes suivants : 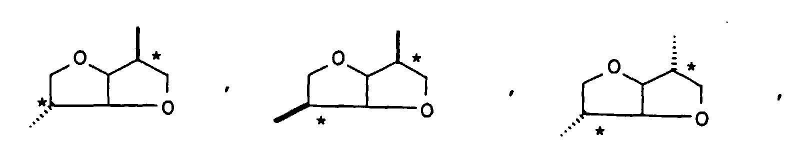 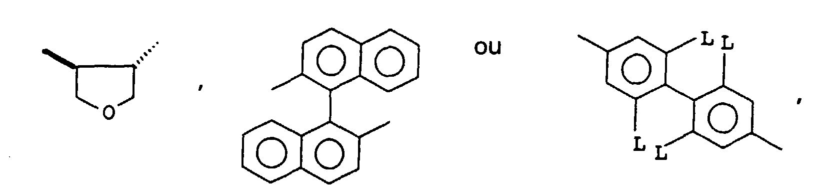 dans lesquels
L représente un groupement alkyle en C₁-C₄, alcoxy en C₁-C₄, un atome d'halogène, COOR, OCOR, CONHR ou NHCOR et R prend la signification citée.

7. Utilisation de composés selon l'une quelconque des revendications 1 à 6 dans des dispositifs d'affichage électro-optiques ou en tant que matière de dopage chirale pour cristaux liquides nématiques ou cholestériques pour la production de couches colorées réfléchissantes.

8. Réseau cholestérique contenant des composés selon l'une quelconque des revendications 1 à 6 sous forme réticulée.
